(19) Europäisches Patentamt
European Patent Office
Office européen des brevets

(11) EP 1 755 570 B1

(12) EUROPEAN PATENT SPECIFICATION

(45) Date of publication and mention
of the grant of the patent:
**13.02.2008 Bulletin 2008/07**

(21) Application number: **05749917.0**

(22) Date of filing: **06.06.2005**

(51) Int Cl.:
*A61K 9/70* (2006.01)

(86) International application number:
**PCT/GB2005/002236**

(87) International publication number:
**WO 2005/120471 (22.12.2005 Gazette 2005/51)**

(54) **TRANSDERMAL DRUG DELIVERY DEVICE COMPRISING EXTENSOR-RELAXOR MEANS**

TRANSDERMALE ARZNEIMITTELABGABEVORRICHTUNG DURCH DEHNUNG/LOCKERUNG

DISPOSITIF DE RELARGAGE DE MEDICAMENT PAR VOIE TRANSDERMIQUE COMPRENANT UN MOYEN EXTENSEUR-RELAXEUR

(84) Designated Contracting States:
**AT BE BG CH CY CZ DE DK EE ES FI FR GB GR HU IE IS IT LI LT LU MC NL PL PT RO SE SI SK TR**

(30) Priority: **05.06.2004 GB 0412590**

(43) Date of publication of application:
**28.02.2007 Bulletin 2007/09**

(73) Proprietor: **Nemaura Pharma Ltd**
**Loughborough**
**Leicestershire LE11 3EH (GB)**

(72) Inventor: **Chowdhury, Dewan Fazlul Hoque**
**Loughborough, Leicestershire LE11 1PN (GB)**

(74) Representative: **Smith, Peter James**
**Serjeants**
**25 The Crescent**
**King Street**
**Leicester LE1 6RX (GB)**

(56) References cited:
WO-A-91/03271        DE-A1- 10 249 853
US-A- 5 370 635      US-A1- 2003 080 085

EP 1 755 570 B1

**Description**

**Technical field**

The invention relates to the administration of drugs to a patient and in particular to their transdermal administration, without the use of a syringe. The term "drug" is used to refer to any biologically active substance that needs to be delivered into the bloodstream of the patient, whether therapeutic or not, for example pharmaceuticals, vaccines and proteins. The patient may be human or animal.

**Background**

The topical administration of drugs was historically limited to the treatment of wounds and other conditions restricted to the external parts of the body. The first transdermal patch based drug delivery system to be marketed was the Transderm Scop™ patch in 1979. Nicotine patches are a more recent example and indicative of the popularity of transdermal systems and their widespread acceptance as drug delivery systems.
Delivery of drugs via the transdermal route has several advantages over more conventional routes such as oral and intravenous or intramuscular. These are listed as follows:

*   Ability to achieve sustained blood drug concentrations, over prolonged periods of up to a week.
*   Predictable pharmacokinetic profiles can be achieved without the sometimes extreme fluctuations that are an inherent part of oral drug delivery.
*   Discontinuation of therapy can be effected immediately upon removal of the transdermal system.
*   Very easy to self-administer compared to intravenous or intramuscular routes, which require qualified nurse or physician to administer.
*   The first-pass effect - metabolism of the drug during its passage through the liver prior to entering the systemic circulation - is avoided.
*   It is a non-invasive mode of drug delivery.
*   Patient activity is not restricted by use of most transdermal systems.
*   Improved patient compliance due to reduced frequency of administration.
*   Drugs inactivated by gastrointestinal enzymes or gastrointestinal pH, (e.g., estrogens, testosterone, nitroglycerin) can be delivered directly into systemic circulation using the transdermal route.
*   Reduced side-effects associated with direct delivery into the systemic circulation, and low delivery dose.

Passive Drug Delivery Mechanisms

The vast majority of marketed transdermal drug delivery systems are based on the passive diffusion of drug molecules through the skin. There have been two main approaches to transdermal systems utilizing passive diffusion, these being the drug-in-adhesive, and the reservoir system. The primary difference between the two systems is that in the reservoir system the drug is loaded in a membrane in the form of a drug reservoir, and must diffuse through the adhesive layer prior to reaching the stratum corneum which it must then penetrate before diffusing through to the capillaries and blood vessels. In the case of the drug-in-adhesive, the drug is loaded into the adhesive layer as well as the reservoir. This eliminates any distance between the drug and the stratum corneum, providing a burst effect and immediate release of drug.
In such systems, passage of drug is dependent on the permeability coefficient which is a function of the resistance to drug diffusion. The resistance itself is a function of the three layers of the skin, the stratum corneum, epidermis and dermis, and the vehicle/polymer within which the drug is contained. This may be shown mathematically as follows:

$$\frac{Dk}{d} = \frac{1}{\text{Resistance (cm}^{-1}\text{ sec)}} = \text{Permeability coefficient (cm sec}^{-1}\text{)}$$

where Dk is the diffusion coefficient of the drug in the stratum corneum;
and d is the thickness of the stratum corneum.
The resistance occurs in series and the total resistance is the sum of the individual resistance offered by the drug loaded vehicle, and each of the three layers of the skin. The total permeability is therefore inversely proportional to the sum of the individual resistances.

Given the relationship and the permeability of the layers of the skin discussed above, it follows that small drug molecules (hydrophilic or lipophilic) are the most likely candidates for delivery via the skin through passive delivery. In order to explore how a broader range of therapeutic molecules may be delivered through the skin it is first necessary to understand the anatomy of the skin, and its barrier properties.

The human skin is made up of three primary layers, the epidermis, dermis and hypodermis. The epidermis is the external surface of the skin, composed primarily of keratinocytes which differentiate into four layers: basal layer, spinos layer, granular layer and surface layer. The surface layer is also known as the stratum corneum and this is the front line defence between the human and its external environment.

The dermis is composed of two layers, the papillary dermis, and reticular dermis. The dermis generally consists of cellular and fibrous components, involved in cell synthesis, and collagen synthesis for tensile strength, and elastic fibre synthesis which imparts deformable properties to the skin.

The hypodermis contains adipose (fat) tissue, and its primary function is to attach dermis to the underlying tissues. Skin appendages originate in the epidermal region, but extend into the dermis. These include hair follicles, sebaceous glands, and arrector pilli muscle (the latter responsible for the erection of hair follicles), and sweat glands.

The skin has numerous functions. These are protection, sensation, heat regulation, formation of a mechanical and immunological defence, synthesis of vitamin D in response to UV exposure, pigmentation for UV protection, and involvement in wound healing.

The skin's protective barrier is composed of a combination of the proteins and lipids that form the stratum corneum, the outermost layer. This layer is continuously shed and regenerated, and provides protection against the entry of water, chemicals, bacteria and fungi. It follows therefore that this is the most important layer with respect to the delivery of drugs transdermally, in that it provides a barrier against the penetration of drugs. The barrier is in the form of 15-20 layers of flat, partially desiccated, dead, keratinised epidermal cells. The thickness of this layer ranges from 10 to 20$\mu$m depending on the region of the body, with the thickest layers being on the palms of the hands and soles of the feet. This barrier is in fact a more formidable barrier to drug delivery than the epithelial barriers of the gastrointestinal, nasal, buccal, vaginal or rectal delivery routes. In addition, at the surface of the skin there are debris, micro-organisms, sebum and other materials, but these present an insignificant barrier to drug penetration.

The stratum corneum barrier is composed of approximately 40% lipids, 40% protein and 20% water. The lipid rich nature precludes the transport of hydrophilic and charged molecules, and facilitates the transport of lipophilic molecules. The structure is analogous to a brick and mortar wall with the hydrated protein making up the bricks, and lipids making up the mortar. Despite the above barrier to drug delivery, once drug molecules cross the stratum corneum, their entry in to the lower layers of the skin and subsequent uptake into the systemic circulation is relatively rapid and unhindered.

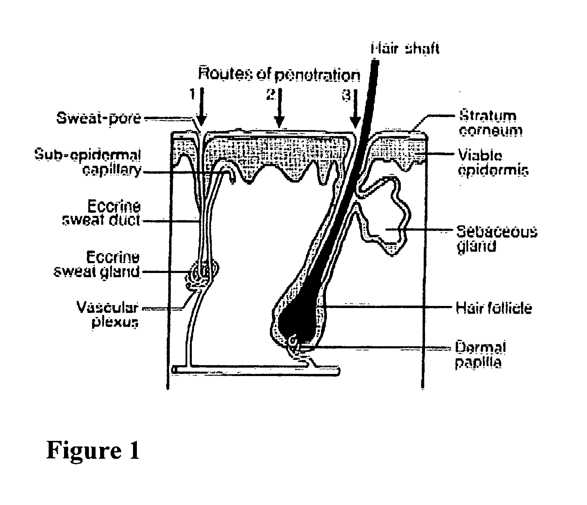

Drugs can penetrate the skin either across the stratum corneum 2, through sweat glands 1, or through hair follicles 3, as indicated in Figure 1.

Active Drug Delivery Mechanisms

Beside the passive methods described above, numerous active methods for delivering drugs through the skin have been investigated. These systems can generally be classified into two categories: by-passing or removal of the stratum corneum, and electrically assisted delivery of drugs. Electrically assisted methods are iontophoresis, phonophoresis, electroporation, use of stress waves, and photomechanical delivery. Follicular delivery allows one to by-pass the stratum corneum, and microscission and microneedle technologies can be used to remove or penetrate the barrier layer. These are further detailed below.

Electrically Assisted Drug Delivery

*Iontophoresis*

Iontophoresis is a non-invasive method of delivering drugs and uses low-level electrical energy in a safe and effective manner. It utilises bipolar electric fields to transport drug molecules across the skin into underlying tissue. The mechanism by which penetration is enhanced has been determined to be due to pore enlargement and/or new pore formation in addition to increased electrochemical potential difference across the skin. There is potential for tissue damage resulting in pain depending on the morphology of the area of application.

*Phonophoresis*

The use of ultrasound (also termed sonophoresis or phonophoresis) in drug delivery was first reported in the 1950's, and currently there are numerous examples of its use to enhance transdermal drug delivery including the delivery of large molecules such as proteins. Ultrasound has been used in the frequency range of 20kHz to 19MHz to increase the permeability of the skin, though it has been demonstrated that frequencies in the lower range of below 100kHz exhibit a higher ability to improve skin permeability.

The general mechanism of action includes ultrasound-mediated thermal effects, transient cavitation, and acoustic streaming. The thermal effects involve the elevation of skin temperature which can enhance the diffusion of molecules through the skin. Efficiency of ultrasound transmission through skin is reduced by attenuation through scatter and absorption, though a few degrees centigrade increase in tissue temperature is achieved, depending on duration of application of the ultrasound. It is thought that the thermal effect is further enhanced by radiation pressure force on the molecules, resulting from the tissue absorbing wave energy, which pushes the molecules in the direction of propagation of the waves. Exposure of the skin to ultrasonics leads to bubble formation which can be mild gas filled bubbles or vapour filled bubbles which violently collapse leading to cavity formation. This can lead to alterations in the skin's structure due to shockwaves created as a result of the collapse. The oscillation of cavitation bubbles leads to liquid streaming, and this is termed acoustic streaming which can also facilitate drug diffusion through the skin.

The main disadvantages are the potential consequences of permanent changes to the skin's structure created by cavitation bubbles.

*Electroportation*

In electroportation a small electric pulse is applied to the skin surface which results in the creation of a transient aqueous pathway through the upper layer of the skin and its protein and lipid membrane. For electroportation to occur the voltage across the skin must reach a few hundred millivolts with an electric field pulse of between $10\mu s$ and 100 ms. Initially upon applying the pulse the membrane becomes charged and after a short period of stability it becomes unstable at which point electroportation occurs.

*Stress waves*

The application of stress waves to the skin using laser has been used to enhance the permeability of the skin to drug molecules. However, it has also been shown that mild heating of the skin prior to using laser induced stress waves (LISW) further significantly enhances the permeability of the skin (2). The mechanism is thought to be an increase in the fluidity of the intercellular lipids resulting in swelling of the corneocytes which allow the laser to form channels for the passage of drug through the skin. The primary complications of this method are the complexity of the procedure and prohibitive equipment costs.

*Photomechanical delivery*

Photomechanical drug delivery involves the use of high pressure gradients to increase skin permeability. The pressure is created by a mechanical stress pulse, generated by a laser. This causes a transient increase in the skin's permeability to drug molecules.

The barrier property of the skin recovers within minutes. It has been demonstrated that macromolecules of up to 40kDa can cross the skin's barrier layer during the transient lapse in its barrier function.

Removing or By-Passing the Skin Surface

*Follicular drug delivery*

Follicular drug delivery utilizes pores associated with skin appendages, such as hair follicle and shaft, and sebaceous glands, to bypass the stratum corneum and allow drugs to penetrate deeper layers of the skin. The cross-sectional area of the follicular route is relatively small, however the rich blood supply associated with skin appendages associated with follicular delivery enhances absorption of the drugs thus enhancing the passage of drugs through the skin.

*Microscission*

This is a technique that creates microconduits through the stratum corneum and underlying tissues, using a combination of momentum transfer and scizing. Momentum is imparted through an ablatory mechanism that utilizes a stream of gas-entrained inert sharp particles that are accelerated towards the skin at an oblique angle. This results in the painless production of micro-holes or conduits on the skin surface. The difference between this and for example use of microneedles is that upon withdrawal of the microneedle the opening that was created closes in on itself. Holes have been created that are in the order of 100-250μm in diameter and 200μm deep, and can be produced repeatedly, rapidly, accurately and painlessly. The accurate control of particle size, flux, carrier gas pressure, area and time of exposure are critical, thus limiting its practical applications. The skin is shown to heal rapidly and does not suffer from adverse events as a result of the ablation.

Microfabrication Technologies

*Microneedle arrays*

The microneedle concept was first conceived in the 1970's, but the first microneedle arrays for increasing skin permeability were developed by Hashmi et al. in the late 1990's with the more widespread availability of fabrication technologies. It has been possible to produce arrays of needles of controlled length, to avoid penetration of the nerves, and sufficiently high strength to penetrate the stratum corneum, thus providing a vehicle for overcoming the skin's protective barrier to enhance drug delivery in a painless manner. They may be used to 'prepare' the skin surface prior to drug delivery via a patch for example. The microneedles may also be used to deliver drugs directly into the skin by interfacing to a drug reservoir, with subsequent control through integrated electronic circuitry and actuation mechanisms.

*MEMS (microelectromechanical systems) syringe*

The MEMS syringe is based on silicon, and soft lithographic techniques. It consists of an array of hollow pointed silicon microneedles and a deformable PDMS (polydimethylsiloxane) reservoir for holding the drug. The design of the system addressed the issue of clog formation caused by shear induced particle sedimentation upon delivery of the drug and has been successfully tested on model skin tissue. The needles are designed to penetrate the skin at depths of up to 200μm, painlessly as there are no nerve endings at these depths, from which they can diffuse into deeper layers of the skin and be absorbed into the blood.
The advantage of this system is that is provides a means of delivering drugs in a dry lyophilised form, which means that storage temperatures do not need to be controlled, hence drugs and vaccines can be widely distributed especially to remote and third world locations.

## The Invention

The invention provides a transdermal drug delivery device as defined in claim 1.
Further, preferred features of the invention are defined in the subclaims.
In this specification, the term "lower" and related terms are used to indicate the side of the transdermal drug delivery device that is intended to be placed in contact with the patient's skin during use. The term "upper" and related terms are used to indicate the opposite side of the device. Such terms are not intended to define the absolute orientation of the device.
When placed in contact with the patient's skin, the device provides active delivery of drugs into the body. The device causes disruption of the skin's biggest barrier to the entry of foreign materials, i.e. the stratum corneum. Moreover, the system causes an expansion of the pores of the skin's follicular route, further enhancing the routes of entry for the said agents. Disruption may also occur to underlying lipid layers causing further enhancement in diffusion of these agents via the skin.
The device has the potential to deliver the following types of drugs and therapeutic agents and molecules:

- Proteins and macromolecules

- Ionic drugs

- Non ionic drugs

- Lipophilic drugs

- Hydrophilic drugs

- Vaccines

The device has the potential to deliver the following formulations:

- Solids - e.g., particles and lyophilised material

- Liquids

- Semi-solids

- Emulsions

- Gels

The device has potential uses in the following therapeutic categories, among others:

- Contraception - e.g., ethinyl estradiol and a novel progesterone, norelgestromin.

- Cancer pain - e.g., Duragesic, a formulation of fentanyl citrate, a potent opioid analgesic, commonly used for chronic cancer pain management.

- CNS - e.g., rotigotine patch for dopamine stimulation required to reduce fluctuating Parkinson's symptoms and selegiline for depression.

- Diabetes - e.g., Insulin

- Hormone replacement and pain related - e.g., Estraderm (estradiol), indicated for the relief of moderate to severe vasomotor symptoms and for the treatment of osteoporosis.

- Cardiovascular - e.g., Nitroderm TTS (nitroglycerin)

- Vaccines - e.g., to elicit an immune response

The device may enhance penetration of the skin through one or more routes including but not restricted to those listed below:

- Transappendageal - the penetrant transverses the stratum corneum via a 'shunt' pathway (e.g. a hair follicle or sweat gland). Given the relative density of hair follicles and sweat glands on the human body, hair follicles are by far the most common route for drug delivery. The mechanical action on the reservoir will create pressure on its contents thus forcing it through the available exits. In this case it will be forced towards the pores and follicles. These pores and follicles will expand in diameter due to the forces exerted on them through extension of the reservoir and its underlying adhesive layer that is in contact with the skin. This may be further enhanced by use of 'vacuum' as described below.

- Transcellular - the permeant crosses the stratum corneum by the most direct route and repeatedly partitions between, and diffuses through, the cornified cells and the extracellular lipid bi-layers. The rate of permeation will be dramatically enhanced as the layer of dead protein/skin cells are disrupted by the physical force exerted on them through extension of the reservoir and its underlying adhesive layer that is in contact with the skin, thus breaking down the biggest barrier to drug entry. This disruption to the stratum corneum may be microscopic or macroscopic or both.

- Lipid bi-layers - Disruption to the underlying lipid bi-layers may cause further enhancement of diffusion of agents through the skin, and stimulation of localised immune type reactions.

### The drawings

Figure 1 is a cross section of the human skin, showing routes for the transdermal delivery of drugs.
Figure 2 is an exploded view showing the general structure of a transdermal drug delivery device according to a preferred embodiment of the invention.

Figures 3 and 4 are schematic cross sections through two alternative embodiments of the reservoir layer in a device according to the invention.

Figures 5 and 6 are schematic plan views of two alternative embodiments of the extensor layer in a device according to the invention.

Figure 7 is a schematic cross section showing the general structure of a transdermal drug delivery device according to a second preferred embodiment of the invention.

## Detailed description of the drawings

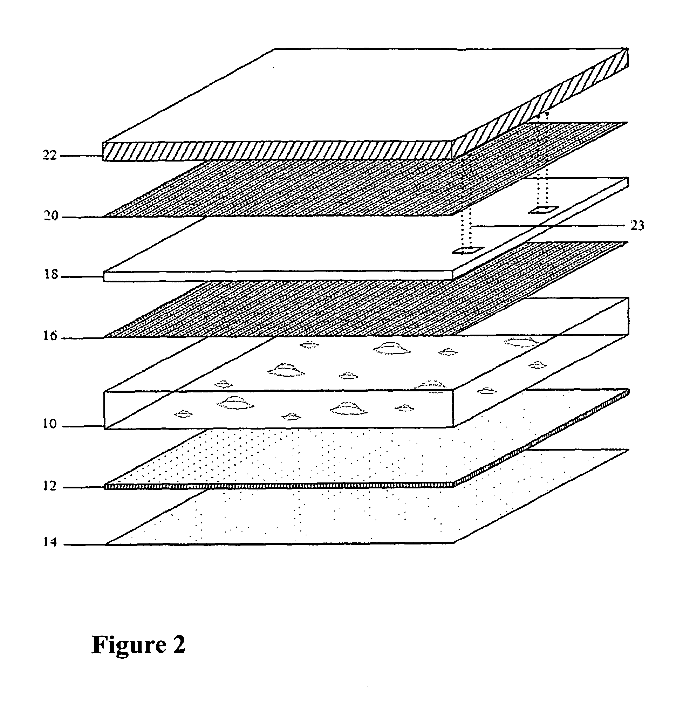

The transdermal drug delivery device illustrated in Figure 2 comprises a reservoir layer 10 that consists of a series of chambers containing one or more drugs or other therapeutic molecules in one or more type of formulation. The reservoir layer 10 is flexible and its lower surface is bounded by a resilient membrane 12, which is perforated by pores through which the drug formulation can pass. An adhesive layer 14 is applied to the membrane 12, which is intended to attach the device to the skin of a patient. The adhesive layer 14 must be suitable for removably bonding the membrane 12 to human or animal skin.

A second adhesive layer 16, which may comprise a different adhesive from the layer 14, bonds an upper surface of the reservoir layer 10 to an extensor layer 18. In this embodiment of the invention, the extensor layer 18 is formed as a microelectromechanical (MEMS) device. A third layer of adhesive 20, which may be similar to the second layer 16, bonds the extensor layer to a control layer 22 comprising microelectronic control circuitry for the extensor layer 18. Electrical contacts between the extensor layer 18 and the control layer 22 are indicated schematically by dotted lines 23.

The device operates by the extensor layer 18 alternately extending and relaxing the reservoir layer 10, so that the drug is squeezed out of the chambers in the reservoir layer 10 and through the pores in the resilient membrane 12.

The stretching and relaxation of the reservoir layer 10 leads to stretching and elongation and relaxation of the pores 26 in the base of the reservoir layer 10. The force on the contents of the reservoir leads to the contents being physically forced in the direction of the skin surface and its appendages.

The stretching and relaxation of the reservoir layer 10 leads in turn to stretching and relaxation of the adhesive layer 14 at the base of the reservoir layer 10 that is attached to the skin. This subsequently leads to stretching and relaxation of the skin and its surface layer, the stratum corneum, and pores such as sweat pores and hair follicles. Extension and relaxation of the skin surface results in disruption of the skin surface cells/barrier and enhancement of pore diameters of the appendages, thus enhancing the delivery of the drug or therapeutic agent through the skin into the body.

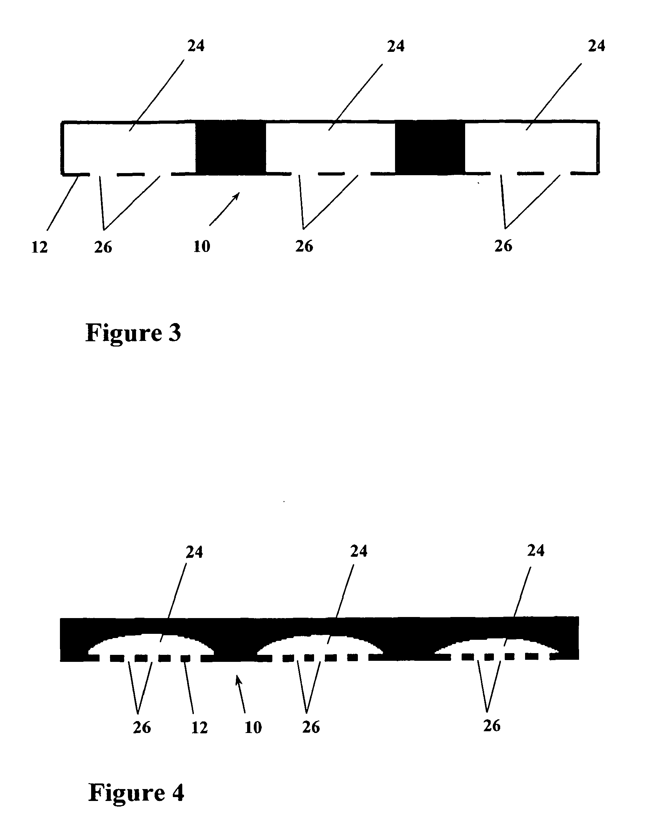

A first example of the reservoir layer 10 is shown schematically in Figure 3. The layer 10 is divided into a number of cuboidal chambers 24, each of which is provided with pores 26 through the portion of the resilient membrane 12 that forms the lower surface of the chamber.

A second example of the reservoir layer 10 is shown schematically in Figure 4. The layer 10 contains a number of domed chambers 24, each of which is provided with pores 26 through the portion of the resilient membrane 12 that forms the lower surface of the chamber.

Each chamber 24 is sufficiently flexible to extend in response to the extensor/relaxor forces placed upon it by the extensor layer 18 of the system. It is also sufficiently rigid and/or externally constrained to allow extension without any significant increase in volume. Preferably, there is a decrease in volume, resulting in pressurised chambers when the device is actuated. The reservoir layer 10 may be composed of numerous large chambers 24 each measuring up to 10mm in diameter, or several hundred smaller chambers 24 each measuring a few micrometers in diameter.

The material of composition of the reservoir chambers 24 may be polymeric, e.g. the Eudragit (Registered Trade Mark) range of pharmaceutical polymers sold by Röhm GmbH, acrylic acid cross-linked polymers, or PDMS (polydimethylsiloxane).

The pores 26 may be formed to be open in the relaxed state of the membrane 12 and will expand and relax upon application of the extensor/relaxor stimulus. The diameter of the pores may be between a few micrometers and 1000 micrometers (1 mm).

Alternatively, the pores may be formed to be constricted, whereby material is not removed during pore creation, thus leading to pores being closed during the relaxed state and open or extended during the extension phase.

The extensor layer 18 may be square, round, or any other shape, which may or may not be the shape and size of the final transdermal patch. It may be located above, below or to one or more edges of the reservoir layer 10.

The degree of extension and relaxation may be ≤0.1% of the total nominal dimensions of the system, or up to about 200% of the total nominal dimensions of the system, the upper limit being determined by the extensibility of the human or animal skin to which the device is to be attached. The frequency of extension and relaxation may be between one cycle per 300 seconds and 1000 cycles per second.

Several factors are inter-related in terms of effect on drug delivery, e.g., degree of pore extension per extensor cycle, and frequency of extensor actuation, and the physical and chemical properties of the formulation in which the drug is contained.

Prolonged extension, i.e., prolonged obstruction free pathway between reservoir and skin pore, may be beneficial e.g., where a low viscosity, solution of low interfacial tension is used to formulate the drug, which is deposited in the reservoir of the device, from which it readily exits upon extensor actuation.

Rapid extension and relaxation may be beneficial for example where a high viscosity formulation is used to incorporate the drug prior to depositing in the reservoir of the delivery device. In this instance the mechanical pressure may be more important in facilitating delivery into the pores of the skin from where the drug may gradually diffuse into the blood circulation.

As previously described, the device according to the invention provides three possible modes of drug delivery, namely via pores, through disruption of the dead protein cells of the stratum corneum, and through disruption of the lipid bi-layers. It follows that where there is diffusion of drug through the enlarged pores, where the extensor actuation frequency may be as low as once per 300 seconds, this leaves little scope for delivery through the second and third routes mentioned above, since the actuation of the extensor layer may not have been vigorous enough to cause disruption of either the stratum corneum or the underlying lipid bi-layers at such low frequency.

There may be circumstances where the various modes of delivery may be required to complement each other in order to achieve therapeutic efficacy. This may thus for example, require initial high frequency for a short duration, resulting in the disruption of the stratum corneum and underlying lipid bi-layers, followed by a lower frequency to allow time for enhanced diffusion to then take place through all three routes. The extensor layer may be controlled to operate with any desired pattern of frequency over time.

The extensor layer 18 may be composed of a number of materials including but not limited to polymeric, gelatinous, metallic, synthetic fibre derived and piezoelectric which, when suitably constructed and interfaced to the control layer 22, will respond to a control stimulus to cause extension and relaxation. Preferably, however, tThe extensor layer 18 is preferably fabricated as a microelectromechanical (MEMS) device consisting of a micrometer sized motor or some other mechanical system that will cause extension and relaxation of a layer of material that is interfaced to the reservoir layer 10. One example of a MEMS system that has been found to be particularly suitable uses an electropolymeric material, a thin film of which can be attached to the reservoir layer 14 to act as the extensor layer 18.

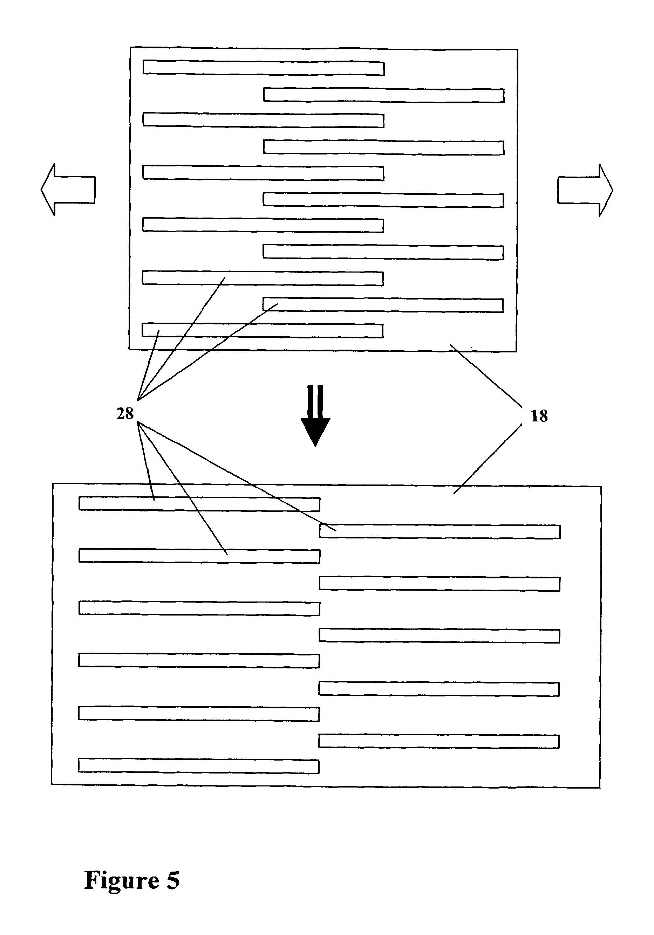

The extension and relaxation caused by the extensor layer may result in elongation along a single axis, as shown in Figure 5. In this example, elements 28 of a microelectromechanical motor slide past one another to extend the rectangular extensor layer 18 in the direction shown by the open arrows. However, other arrangements of motor elements could be used to achieve the same result.

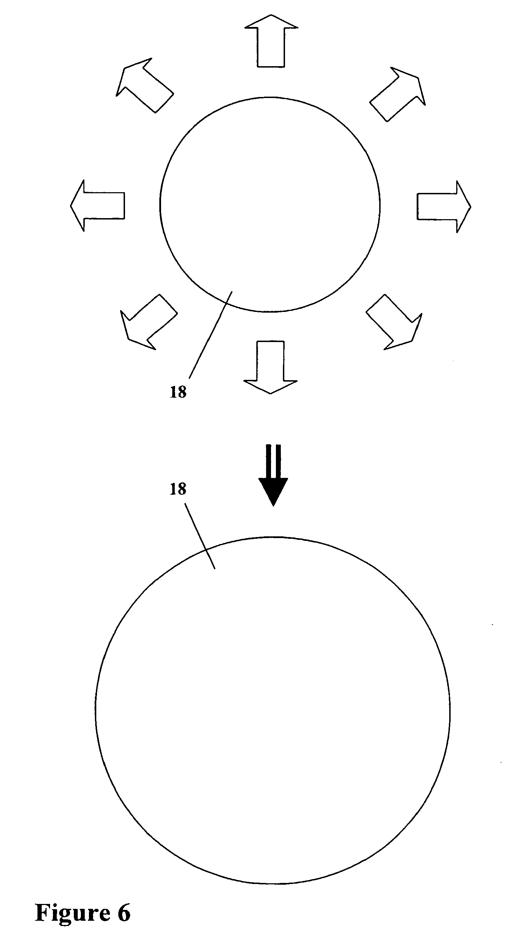

Alternatively, extension and relaxation caused by the extensor layer 18 may be equal in all directions to give equal extension and relaxation of the reservoir layer 10 in all directions, or may be uneven in various directions to give uneven extension and relaxation in various directions. Figure 6 illustrates an example of a circular extensor layer 18 that is extended by a microelectromechanical motor (not shown) equally in all directions, as shown by the open arrows, or comprises a film of electropolymeric material that expands equally in all directions on connection to a power supply.

The control layer 22 will contain appropriate microelectronic circuitry, designed to control the extension and relaxation of the extensor layer 18, in terms of degree and frequency of extension and relaxation. The fabrication of the control layer 22 shall be using standard integrated circuit fabrication technology, and appropriate materials, layout and interface to the extensor layer 18, as will be evident to a skilled person in the field.

The relaxation of the reservoir layer 14 may be passive, i.e. effected by the resilient reservoir layer 14 returning naturally to its relaxed state. Preferably, and especially when the device is to be operated at high frequencies, the extensor layer 18 may be actively controlled to drive the reservoir layer 14 to its relaxed state.

The control layer 22 may also contain a power supply, which may be a standard thin film power supply or may utilise polymer film cell technology, providing power from a thin film of polymer or other thin film material.

The extensor layer 18 may be configured so as to actuate the chambers 24 of the reservoir layer 10 either collectively, or in groups, or individually through control over the actuation of the extensor layer motions by the control layer 22, and appropriate interfacing of the extensor layer 18 to the reservoir layer 10 and its chambers 24.

Actuation of the device may be instantaneous, delayed, or intermittent over a period of hours or days.

Appropriate pharmaceutically acceptable and compatible adhesives shall be used in the adhesive layers 16,20 to interface the various layers and components and in the adhesive layer 14 that is to be in contact with the skin. The adhesive layers 14,16,20, in particular the layer 14 in contact with the skin, shall be sufficiently strong to withstand lateral extension forces, and be flexible enough to be able to relax back to their original state.

Passage of drugs and other therapeutic molecules through the adhesive layer 14 must not be of any significant impact on the overall functioning of the system. Extension of the reservoir layer 10 will result in the extension of the adhesive layer 14 too, thus resulting in thinning of the adhesive layer 14 and further minimizing any effect on the passage of drugs through the adhesive layer. Pores may be created in the adhesive layer 14. If they are directly opposite the pores 26 in the reservoir layer 10, they will provide an obstruction free path for the flow of materials.

Given that the uppermost layer of the skin, the stratum corneum, consists of dead cells, it is conceivable that upon repeated cycles of extension and relaxation, the adhesion to the skin may be lost in certain areas due to the complete removal of dead skin cells from the stratum corneum, in particular where the degree of extension is large.

In such circumstances a beneficial feature would be an increase in the surface roughness of the porous resilient membrane 12, such that the area of contact with the surface of the skin is increased. This rough surface could be uniform or non-uniform and may even provide anchorage points on the skin surface, thus making localised loss of adhesion less likely or absent altogether.

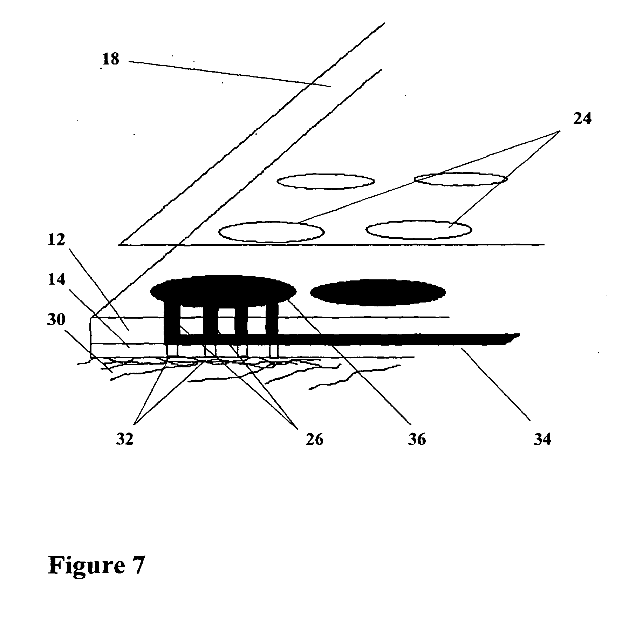

A further enhancement of the device is illustrated in Figure 7, in contact with a patient's skin 30. The enhanced device includes additional features in the reservoir layer 10 that may further increase drug uptake through the appendages of the skin and also enhance diffusion.

In this embodiment of the invention, the resilient membrane 12 includes large pores 26 with slightly rigid walls. A thinner, collapsible membrane 36 seals the pores 26 from the chamber 24 containing the drug. The collapsible membrane 36 is strong enough not to be breached upon application of the extensor and relaxor stimulus to the reservoir layer 10. Pores 32 in the adhesive layer 14 are aligned with the pores 26 in the resilient membrane 12.

Micro-channels 34 lead from the pores 26,32 to a pump or some other vacuum creating device (not shown), which may be micro-fabricated. Activation of the pump will cause the evacuation of the pores 26,32 and thus a reduction in pressure in the pores. The results will be collapse of these pores 26,32 and to an extent, collapse of the pores in the skin itself that have been widened by the flexor/relaxor motion of the device.

Once a threshold level of vacuum is reached, the collapsible membrane 36 will collapse, resulting in the drug filling both the pore cavities 26,32 in the device leading out of the reservoir layer 10, and the pores in the skin 30 that have partially collapsed towards the upper surface. The speed of uptake of the drug or therapeutic agent will be dramatically enhanced as a result.

In the enhanced device as described and illustrated, the collapsible membrane 36 can only be ruptured once, therefore this device is most suitable for administering a single dose of the drug. Alternative means of providing a temporary seal between the chambers 24 and the pores 26 may be provided. One such alternative would be a micro-valve, which remains closed until the pressure difference across it exceeds a threshold and then opens to release the drug from the chamber 24. When the pump is switched off and the pressure difference falls again, such a valve may close again, allowing the device to be re-used. Thereby the enhanced device can be used to deliver the drug to the patient intermittently over an extended period.

**Claims**

1. A transdermal drug delivery device comprising:

   a reservoir layer (10), which comprises one or more chambers (24) for containing a drug, a lower surface of the reservoir layer (10) being bounded by a resilient membrane (12) perforated by pores (26) through which the drug may be delivered from the chambers (24); and
   extensor means (18), which actuates on receipt of a control stimulus to deform the reservoir layer (10) between a first state in which the pores (26) are reduced in size and a second state in which the pores (26) are enlarged.

2. A transdermal drug delivery device according to claim 1, wherein the chambers (24) are more compressed in the second state than in the first state.

3. A transdermal drug delivery device according to claim 1 or claim 2, wherein the first state is a relaxed state of the reservoir layer (10).

4. A transdermal drug delivery device according to any preceding claim, wherein the extensor means (18) is connected to the reservoir layer (10) in such a manner that when the extensor means (18) is actuated, it stretches the reservoir layer (10).

5. A transdermal drug delivery device according to claim 4, wherein when the extensor means (18) is actuated, it stretches the reservoir layer (10) along a single axis.

6. A transdermal drug delivery device according to claim 4, wherein when the extensor means (18) is actuated, it stretches the reservoir layer (10) along two orthogonal axes.

7. A transdermal drug delivery device according to any preceding claim, wherein the extensor means (18) is attached to an upper surface of the reservoir layer (10).

8. A transdermal drug delivery device according to any preceding claim, wherein the extensor means (18) is formed as a microelectromechanical (MEMS) device.

9. A transdermal drug delivery device according to any preceding claim, further comprising control means (22) for providing the control stimulus to the extensor means (18).

10. A transdermal drug delivery device according to claim 9, wherein the control means (22) is a microelectronic control, which can control the actuation of the extensor means (18) in accordance with a predefined drug delivery regime.

11. A transdermal drug delivery device according to any preceding claim, further comprising a layer of adhesive (14) on the resilient membrane (12), through which the drug can pass, the adhesive (14) being suitable for adhering the device to the skin (30) of a patient.

12. A transdermal drug delivery device according to claim 11, wherein pores (32) are formed in the layer of adhesive (14) to assist the passage of the drug through the layer.

13. A transdermal drug delivery device according to any preceding claim, further comprising:

   a seal (36) between the chambers (24) of the reservoir layer (10) and the pores (32) through the resilient membrane (14);
   a micro-pump connected to the pores (32) through the resilient membrane (14), the micro-pump being capable of reducing the air pressure in the pores (32) sufficiently to break the seal (36) and thereby release the drug from the chambers (24) into the pores (32).

14. A transdermal drug delivery device according to claim 12, wherein the seal (36) is formed by a collapsible membrane, which ruptures when the pressure difference across it exceeds a threshold.

15. A transdermal drug delivery device according to claim 12, wherein the seal is formed by a valve, which opens only when the pressure difference across it exceeds a threshold.

**Patentansprüche**

1. Transdermale Arzneimittelabgabevorrichtung, mit
   einer Reservoirlage (10), welche eine oder mehrere Kammern (24) zur Aufnahme eines Medikaments aufweist,
   einer unteren Oberfläche der Reservoirlage (10), welche über eine elastische Membran (12), welche durch Poren (26) perforiert ist, durch welche das Medikament aus den Kammern (24) abgegeben werden kann, befestigt ist, und
   einer Extensoreinrichtung (18), welche bei Empfang eines Steueranreizes aktiv wird, um die Reservoirlage (10) zwischen einem ersten Zustand, in welchem die Poren (26) in der Größe reduziert sind und einem zweiten Zustand, in welchem die Poren (26) vergrößert sind, zu verformen.

2. Transdermale Arzneimittelabgabevorrichtung nach Anspruch 1, wobei die Kammern (24) im zweiten Zustand mehr komprimiert sind, als im ersten Zustand.

3. Transdermale Arzneimittelabgabevorrichtung gemäß Anspruch 1 oder Anspruch 2, wobei der erste Zustand ein entspannter Zustand der Reservoirlage (10) ist.

4. Transdermale Arzneimittelabgabevorrichtung nach einem der vorhergehenden Ansprüche, wobei die Extensoreinrichtung (18) mit der Reservoirlage (10) derart verbunden ist, dass sie die Reservoirlage (10) streckt, wenn die Extensoreinrichtung (18) betätigt wird.

5. Transdermale Arzneimittelabgabevorrichtung nach Anspruch 4, wobei, wenn die Extensoreinrichtung (18) betätigt wird, sie die Reservoirlage (10) entlang einer einzelnen Achse streckt.

6. Transdermale Arzneimittelabgabevorrichtung nach Anspruch 4, wobei, wenn die Extensoreinrichtung (18) betätigt wird, sie die Reservoirlage (10) entlang zweier orthogonaler Achsen streckt.

7. Transdermale Arzneimittelabgabevorrichtung nach einem der vorhergehenden Ansprüche, wobei die Extensoreinrichtung (18) an einer oberen Oberfläche der Reservoirlage (10) angebracht ist.

8. Transdermale Arzneimittelabgabevorrichtung nach einem der vorhergehenden Ansprüche, wobei die Extensoreinrichtung (18) als eine mikroelektromechanische Vorrichtung (MEMS) ausgebildet ist.

9. Transdermale Arzneimittelabgabevorrichtung nach einem der vorhergehenden Ansprüche, mit einer Steuereinrichtung (22), um den Steueranreiz für die Extensoreinrichtung (18) abzugeben.

10. Transdermale Arzneimittelabgabevorrichtung nach Anspruch 9, wobei die Steuereinrichtung (22) eine mikroelektronische Steuerung ist, welche die Betätigung der Extensoreinrichtung (18) gemäß einem vordefinierten Arzneimittelabgabeprogramm regeln kann.

11. Transdermale Arzneimittelabgabevorrichtung nach einem der vorhergehenden Ansprüche, mit einer Lage eines Adhäsivs (14) auf der elastischen Membran (12), durch welche das Arzneimittel hindurch gehen kann, wobei das Adhäsiv (14) geeignet ist, die Vorrichtung an der Haut (30) eines Patienten anzuheften.

12. Transdermale Arzneimittelabgabevorrichtung nach Anspruch 11, wobei in der Lage des Adhäsivs (14) Poren (32) gebildet sind, um den Durchgang des Arzneimittels durch die Lage zu unterstützen.

13. Transdermale Arzneimittelabgabevorrichtung nach einem der vorhergehenden Ansprüche, mit
einer Dichtung (36) zwischen den Kammern (24) der Reservoirlage (10) und den Poren (32) durch die elastische Membran (14),
einer Mikropumpe, welche mit den Poren (32) durch die elastische Membran (14) verbunden ist, wobei die Mikropumpe geeignet ist, den Luftdruck in den Poren (32) ausreichend zu reduzieren, um die Dichtung (36) aufzulösen und hierdurch das Arzneimittel aus den Kammern (24) in die Poren (32) abzugeben.

14. Transdermale Arzneimittelabgabevorrichtung nach Anspruch 12, wobei die Dichtung (36) durch eine kollabierbare Membran gebildet ist, welche reißt, wenn die Druckdifferenz hierüber einen Grenzwert überschreitet.

15. Transdermale Arzneimittelabgabevorrichtung nach Anspruch 12, wobei die Dichtung durch ein Ventil gebildet ist, welches sich nur öffnet, wenn die Druckdifferenz hierüber einen Grenzwert überschreitet.

**Revendications**

1. Dispositif de relargage de médicament par voie transdermique, comprenant :

une couche réservoir (10), qui comprend une ou plusieurs chambres (24) pour contenir un médicament, une surface inférieure de la couche réservoir (10) étant liée par une membrane élastique (12) perforée de pores (26) à travers lesquels le médicament peut être largué à partir des chambres (24) ; et
un moyen extenseur (18), lequel s'actionne à la réception d'un stimulus de commande pour déformer la couche réservoir (10) entre un premier état dans lequel les pores (26) sont réduits en taille et un second état dans lequel les pores (26) sont agrandis.

2. Dispositif de relargage de médicament par voie transdermique selon la revendication 1, dans lequel les chambres (24) sont plus comprimées dans le second état que dans le premier état.

3. Dispositif de relargage de médicament par voie transdermique selon la revendication 1 ou la revendication 2, dans lequel le premier état est un état relâché de la couche réservoir (10).

4. Dispositif de relargage de médicament par voie transdermique selon l'une quelconque des revendications précédentes, dans lequel le moyen extenseur (18) est connecté à la couche réservoir (10) de sorte que quand le moyen extenseur (18) est actionné, il étire la couche réservoir (10).

5. Dispositif de relargage de médicament par voie transdermique selon la revendication 4, dans lequel quand le moyen extenseur (18) est actionné, il étire la couche réservoir (10) le long d'un seul axe.

6. Dispositif de relargage de médicament par voie transdermique selon la revendication 4, dans lequel quand le moyen extenseur (18)est actionné, il étire la couche réservoir (10) le long de deux axes orthogonaux.

7.  Dispositif de relargage de médicament par voie transdermique selon l'une quelconque des revendications précédentes, dans lequel le moyen extenseur (18) est fixé à une surface supérieure de la couche réservoir (10).

8.  Dispositif de relargage de médicament par voie transdermique selon l'une quelconque des revendications précédentes, dans lequel le moyen extenseur (18) est formé en tant qu'un microsystème électromécanique (MEMS).

9.  Dispositif de relargage de médicament par voie transdermique selon l'une quelconque des revendications précédentes, comprenant en outre un moyen de commande (22) pour fournir le stimulus de commande au moyen extenseur (18).

10. Dispositif de relargage de médicament par voie transdermique selon la revendication 9, dans lequel le moyen de commande (22) est une commande micro-électrique, laquelle peut commander l'actionnement du moyen extenseur (18) selon un schéma de largage de médicament prédéfini.

11. Dispositif de relargage de médicament par voie transdermique selon l'une quelconque des revendications précédentes, comprenant en outre une couche d'adhésif (14) sur la membrane élastique (12), à travers laquelle le médicament peut passer, l'adhésif (14) étant adapté pour faire adhérer le dispositif sur la peau (30) d'un patient.

12. Dispositif de relargage de médicament par voie transdermique selon la revendication 11, dans lequel les pores (32) sont formés dans la couche d'adhésif (14) pour faciliter le passage du médicament à travers la couche.

13. Dispositif de relargage de médicament par voie transdermique selon l'une quelconque des revendications précédentes, comprenant en outre :

    un joint (36) entre les chambres (24) de la couche réservoir (10) et les pores (32) à travers la membrane élastique (14) ;
    une micro-pompe connectée aux pores (32) à travers la membrane élastique (14), la micro-pompe ayant la capacité de réduire la pression d'air dans les pores (32) de manière suffisante afin de briser le joint (36) et libérer de cette manière le médicament depuis les chambres (24) dans les pores (32).

14. Dispositif de relargage de médicament par voie transdermique selon la revendication 12, dans lequel le joint (36) est formé par une membrane pliable, laquelle se rompt quand la différence de pression d'un côté à l'autre de celle-ci excède un seuil.

15. Dispositif de relargage de médicament par voie transdermique selon la revendication 12, dans lequel le joint est formé par une valve, laquelle s'ouvre seulement quand la différence de pression d'un côté à l'autre de celle-ci excède un seuil.

# Figure 1

**Figure 2**

**Figure 3**

**Figure 4**

**Figure 5**

18

18

**Figure 6**

18    24    12    14    30    32    26    36    34

**Figure 7**